# EUROPEAN PATENT APPLICATION

(11) **EP 2 455 074 A1**
(43) Date of publication of application: **23.05.2012**
(21) Application number: 11187973.0
(22) Date of filing: 04.11.2011
(51) Int. Cl.: A61K 31/05, A61K 47/10, A61K 9/00

(54) **Topical pharmaceutical compositions of flurbiprofen and methyl salicylate**

(30) Priority: 08.11.2010 TR 201009220
(71) Applicant: Sanovel Ilac Sanayi ve Ticaret A.S., 34398 Istanbul (TR)
(72) Inventor: Cifter, Ümit, 34398 Istanbul (TR); Türkyilmaz, Ali, 34398 Istanbul (TR); Akalin, Nur Pehlivan, 34398 Istanbul (TR); Önder, Ramazan, 34398 Istanbul (TR); Öner, Levent, Ankara (TR)
(74) Representative: Sevinç, Erkan

(57) **Abstract**

The present invention relates to topical pharmaceutical compositions comprising flurbiprofen or a pharmaceutically acceptable salt thereof and methyl salicylate. More specifically, the invention relates to topical pharmaceutical compositions of flurbiprofen and methyl salicylate characterized in that said composition comprises dimethyl sulfoxide and one or more gelling agent. Furthermore, the invention relates to process for preparing the said topical pharmaceutical compositions and its use for the treatment of pain and inflammatory symptoms associated with muscle-skeletol system, joint and soft-tissue disorders.

## Description

### Field of Invention

The present invention relates to topical pharmaceutical compositions comprising flurbiprofen or a pharmaceutically acceptable salt thereof and methyl salicylate. More specifically, the invention relates to topical pharmaceutical compositions of flurbiprofen and methyl salicylate characterized in that said composition comprises dimethyl sulfoxide and one or more gelling agent. Furthermore, the invention relates to process for preparing the said topical pharmaceutical compositions and its use for the treatment of pain and inflammatory symptoms associated with muscle-skeletol system, joint and soft-tissue disorders.

### Background of Invention

Flurbiprofen is a well known, propionic acid derivative, also known as NSAID (non-steroidal anti-inflammatory drug), with the analgesic and anti-inflammatory activities it possesses. It is used in muscle-skeletal and joint disorders such as ankylosing spondylitis, osteoarthritis and rheumatoid arthritis, in soft-tissue disorders such as sprains and strains and for postoperative pains. Its chemical structure is illustrated with Formula I given below.

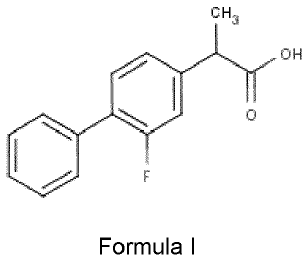

Flurbiprofen is mostly administrated orally. One disadvantage of the oral administration of flurbiprofen comprising compositions is that the patient is likely to experience unpleasant side effects, including gastrointestinal irritation.

The use of flurbiprofen in treating local pains and inflammations may cause a problem especially for those who have gastrointestinal system disorders. It is possible to develop various locally-administrable topical forms of flurbiprofen, in order to avoid the systemic side-effects thereof. The skin absorption rate of the relevant product to be used in topical applications, however, is quite significant. Enhancing the absorption rate both provides ease of application and increases the molecule's efficiency.

Methyl salicylate is a natural product having an esther structure which is shown in Formula II, given below. It is used to relief the aches and pains in mostly muscle-skeletal, joint and soft-tissue disorders.

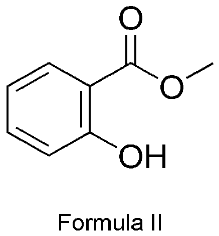

Even if there is not any problem associated with the absorption of methyl salicylate, which is the other active ingredient used in the formulation, the requirement is obvious to increase the rate of absorption particularly during acute situations.

In prior art, there are also several patents which disclose flurbiprofen or methyl salicylate in several pharmaceutical dosage forms but none of them disclose the use of gelling agents and dimethyl sulfoxide in combination with them to have new and improved topical pharmaceutical composition.

US 4 545 992, disclose a pharmaceutical preparation for external use which comprises a solution of a non-steroid analgesic agent which is dissolved in at least one adjuvant selected from the group consisting of peppermint oil, methyl salicylate, ethyl salicylate and monoglycol salicylate in an amount at least sufficient to dissolve said analgesic agent and bases for external use but it is silent about the penetration problems and the use of dimethyl sulfoxide and gelling agents in combination with flurbiprofen and methyl salicylate.

Particularly in acute disorders, there arises the need of enhancing the absorption rate at the site of administration. For instance, during which local pains associated with injuries in sportive events are to be urgently alleviated, it becomes necessary to apply local anesthesia to the relevant site.

The flurbiprofen containing compositions described above have at least one disadvantage in that the amount of drug delivered transdermally is not maximized. Accordingly, there exists need for a percutaneous delivery system for the drug flurbiprofen and methyl salicylate which optimizes the delivery of them through the skin.

It is therefore an object of the present invention to provide a topical pharmaceutical composition of flurbiprofen and methyl salicylate which is more effective for purposes of percutaneous delivery of them through the skin.

### Summary of the Invention

The present invention relates to an easily applicable flurbiprofen and methyl salicylate topical pharmaceutical composition, which overcomes the above described problems in prior art and have additive advantages over them.

Accordingly, the main object of the present invention is to increase the rate of percutaneous penetration, thereby shortening the time period in which the active agents exert their effect.

The rate of percutaneous penetration of said combination is enhanced with the dimethyl sulfoxide and gelling agents it contains.

Another object of the present invention is to obtain a stable topical pharmaceutical composition of flurbiprofen and methyl salicylate during the shelf-life and to exhibit high safety when applied to skin.

A further object of the present invention is to obtain a formulation with local anesthetic effect, with the menthol used in said combination stimulating the receptors by which the cold sensation is perceived.

Accordingly, a topical pharmaceutical composition, more specifically a gel composition has been developed to achieve all objects referred above.

In a preferred embodiment according to the present invention, said novelty is realized with flurbiprofen or a pharmaceutically acceptable salt thereof and methyl salicylate characterised in that said composition comprise dimethyl sulfoxide and one or more gelling agent.

In another preferred embodiment according to the present invention, the gelling agents are selected from the group comprising hydroxypropyl cellulose, hydroxyethyl cellulose, methyl cellulose, carboxymethyl cellulose, carbomer, carbomer copolymers, gelatin, aluminum monostearat, dextrin, sodium alginate, pectin and mixtures thereof. Preferably the gelling agents are hydroxypropyl cellulose and carbomer or mixtures thereof.

According to a preferred embodiment, the weight ratio of hydroxypropyl cellulose and carbomer is from 10:1 to 1:10 by weight, preferably it is from 4:1 to 1:4 by weight of the total composition.

According to a preferred embodiment of the present invention the amount of dimethyl sulfoxide is from 0.5 to 30.0% by weight of the total composition, preferably it is 5.0 to 20.0% by weight of the total composition.

According to a preferred embodiment of the present invention, the topical pharmaceutical composition is in the form of gel, ointment, cream, spray or lotion, preferably it is in the form of gel.

According to a preferred embodiment of the present invention, the topical pharmaceutical composition further comprises menthol, wherein the amount of menthol is between 0.10 to 15.0% by weight of the total composition, preferably it is 1.0 to 10.0% by weight of the total composition.

According to a preferred embodiment of the present invention, the topical pharmaceutical composition, further comprises surface active agents, viscosity enhancers, dissolving solvents, preservatives, antioxidants and their mixtures

According to a preferred embodiment, the present invention comprise polysorbate as the surface active agent, wherein the amount of polysorbate is 0.05 to 5.0% by weight of the total composition, preferably it is 0.10 to 3.0% by weight of the total composition.

According to a preferred embodiment, the present invention comprise glycerin as the viscosity enhancer, wherein the amount of glycerin is 1.0 to 50.0% by weight of the total composition, preferably it is 5.0 to 15.0% by weight of the total composition.

In a further preferred embodiment of the present invention, said topical pharmaceutical composition comprise the following;

| | | |
|---|---|---|
| a. | flurbiprofen | 0.5 to 15.0% by weight |
| b. | methyl calicylate | 1.0 to 20.0% by weight |
| c. | hydroxypropyl cellulose | 0.05 to 10.0% by weight |
| d. | carbomer | 0.01 to 5.0% by weight |
| e. | dimethyl sulfoxide | 0.5 to 30.0% by weight |
| f. | polyethylene glycol | 1.0 to 50.0% by weight |
| g. | menthol | 0.1 to 15.0% by weight |
| h. | polysorbate | 0.05 to 5.0% by weight |
| i. | glycerin | 1.0 to 50.0% by weight |
| j. | purified water | 1.0 to 30.0% by weight |
| k. | ethyl alcohol | 1.0 to 50.0% by weight |
| I. | NaOH / HCl | pH (5.5 ±1.0) |

Another aspect of the present invention provides a method for preparing the topical pharmaceutical composition according to the present invention, this method comprising the steps of;
a. adding carbomer, purified water, glycerin, polyethylene glycol, dimethyl sulfoxide and swelling this mixture under stirring so as to yield the first mixture,
b. adding NaOH / HCl to the first mixture to adjust the pH and stirring,
c. adding and dissolving flurbiprofen and methyl calicylate into alcohol in a separate container and then adding menthol, polysorbate and hydroxypropyl cellulose into this mixture so as to give the second mixture,
d. adding the second mixture into the first mixture under stirring

According to another preferred embodiment of the present invention, the topical pharmaceutical composition is used in the treatment of pain and inflammatory symptoms associated with muscle-skeletol system, joint and soft-tissue disorders.

Further advantages and embodiments of the present invention will become apparent from the following description

### Detailed Description of Invention

According to the present invention, a novel formulation with anti-inflammatory and analgesic activities is obtained surprisingly, which is rapidly absorbed and gives local anesthetic effect.

The flurbiprofen useful in accordance with this invention includes the pharmaceutically acceptable salts and esters of flurbiprofen, and further includes the conventionally used racemic mixture which comprises the S- and R- enantiomers of flurbiprofen. The topical pharmaceutical compositions of the invention comprise from 0.5 to 15.0% flurbiprofen, preferably from 2.0 to 10.0%, more preferably from 3.0 to 7.0%, and most preferably about 5.0% by weight of the total composition.

The topical pharmaceutical compositions of the invention comprise from 1.0 to 20.0% methyl salicylate, preferably from 5.0 to 15.0%, more preferably about 10.0% by weight of the total composition.

The topical pharmaceutical compositions of the invention comprise from 0.5 to 30.0% dimethyl sulfoxide, preferably from 5.0 to 20.0%, more preferably from 10.0 to 15.0%, most preferably about 13.33% by weight of the total composition. Dimethyl sulfoxide helps the composition of the present invention to improve and enhance the penetrating and spreading properties through the skin. It also helps to carry out other components easily and without damaging the membranes into biological sytem. Accordingly, dimethyl sulfoxide is used as a topical analgesic, a vehicle for topical application of pharmaceuticals, as an anti-inflammatory and an antioxidant. These properties, surprisingly is found to have synergistic effect over flurbiprofen and methyl salicylate activity to have better treatment over the pain and inflammatory symptoms associated with muscle-skeletol system, joint and soft-tissue disorders.

The topical pharmaceutical compositions of the invention comprise from 0.01 to 15.0% gelling agents, preferably from 0.05 to 10.0% by weight. Suitable gelling agents may comprise but not limited to hydroxypropyl cellulose, hydroxyethyl cellulose, methyl cellulose, carboxymethyl cellulose, carbomer, carbomer copolymers, gelatin, aluminum monostearat, dextrin, sodium alginate, pectin and mixtures thereof. Preferably the gelling agents are hydroxypropyl cellulose and carbomer or mixtures thereof. Surprisingly it is found that when the weight ratio of hydroxypropyl cellulose to carbomer is from 10:1 to 1:10 by weight, preferably from 4:1 to 1:4, more preferably 2:1 to 1:2 by weight; it enhance the penetration properties of the formulation in combination with dimethyl sulfoxide. Accordingly, hydroxypropyl cellulose and carbomer help to obtain the viscosity in a stable level to enhance the penetration of the topical composition and their stabilizer and emulsifier property is also help them to show this effect easily.

Furthermore, the topical pharmaceutical compositions of the invention comprise menthol from 0.10 to 15.0%, preferably from 1.0 to 10.0%, more preferably about 5.0% by weight of the total composition. Menthol used in the formulation gives anesthetic effect at the side of administration as a result of stimulating the receptors by which cold sensation is perceived. Methyl salicylate and menthol provide significant relief of pain associated with mild to moderate muscle strain in adult patients. Also menthol helps to mask the bad odour of the methyl salicylate to obtain a good patient compliance when applying to skin.

The pharmaceutical compositions according to the present invention may also comprise one or more pharmaceutically acceptable excipients. Such proper pharmaceutically acceptable excipients comprise, but are not limited to surface active agents, viscosity enhancers, dissolving solvents, preservatives, antioxidants and their mixtures.

Suitable surface active agents may comprise but not limited to polysorbate, menthol, dimethyl sulfoxide, diethanolamine, glyceryl monostearat, oleic acid, sodium lauril sulfate, propylene glycol, polyethylene glycol succinate and mixtures thereof. Preferably the surface active agent is polysorbate 20, polysorbate 40, polysorbate 60, polysorbate 80 and mixtures thereof. The most preferred one is polysorbate 80. The amount of polysorbate 80 is from 0.05 to 5.0%, preferably 0.1 to 3.0%, more preferably about 1.0% by weight of the total composition. Polysorbate has also a stabilisator effect when it is used in these amounts and helps the formulation to be stable over the shelf life.

Suitable viscosity enhancers may comprise but not limited to glycerin, pullulan, dextran, cellulose and derivatives, chitosan, carbomer and mixtures thereof. Preferably the viscosity enhancer is glycerin and the amount is from 1.0 to 50.0%, more preferably from 5.0 to 15.0% by weight of the total composition. These amounts of glycerin improve the spreading properties and minimize any balling up or drying of the compositions of the present invention when it is rubbed on the skin.

Suitable dissolving solvents may comprise but not limited to ethyl alcohol, polyethylene glycol, glycerin, isopropyl alcohol and purified water. Preferably ethyl alcohol, polyethylene glycol and purified water are used. Ethyl alcohol is also utilized as a microbiological preservative.

Suitable preservatives may comprise but not limited to methylparaben, propylparaben, sodium and potassium benzoate, imidurea, monothioglicol, phenyl mercuric derivatives, sodium sulfate, sodium meta bisulfate, potassium sorbate or mixtures thereof. The amount of the preservatives is from 0.0 to 2.0% by weight of the total composition.

Suitable antioxidants may comprise but not limited to alpha tocopherol, butyl hydroxy anisole, butyl hydroxyl toluene, monothioglicol, sodium meta bisulfate, potassium meta bisulfate and mixtures thereof. The amount of the antioxidants is from 0.0 to 2.0% by weight of the total composition.

The pharmaceutical compositions according to the present invention provides spreadable, semi-solid and jelly-like gel compositions of flurbiprofen or a pharmaceutically acceptable salt thereof and methyl salicylate. However, the topical compositions of the invention may also take the form of ointment, cream, spray or lotion

Accordingly, the present invention may be used for treating pain and inflammatory symptoms associated with muscle-skeletol system, joint and soft-tissue disorders.

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

### Example

| Content | amount (%) (w/w) |
|---|---|
| Flurbiprofen | 5.00 |
| Methyl salicylate | 10.00 |
| Hydroxypropyl cellulose H | 1.33 |
| Carbomer 940 | 0.83 |
| Dimethyl sulfoxide | 13.33 |
| Polyethylene glycol 400 | 11.67 |
| Menthol | 5.00 |
| Polysorbate 80 | 1.00 |
| Glycerin | 8.33 |
| Purified water | 13.33 |
| Ethyl alcohol | 30.17 |
| NaOH / HCL | pH 5.5 ±1.0 |

Carbomer 940, glycerin, polyethylene glycol 400 and dimethyl sulfoxide is added into purified water under stirring and the mixture is swollen by keeping it stirred for about 60 min and homogenised. Thus, the first mixture is obtained, and the pH is adjusted with NaOH / HCL. Flurbiprofen and methyl salicylate is dissolved in a separate container in ethyl alcohol. Thus, the second mixture is obtained. Then menthol, hydroxypropyl cellulose H and polysorbate 80 is added into the second mixture under stirring for about 90 min and then homogenised. The second mixture is added to the first mixture under stirring for about 10 min and filled up with ethyl alcohol. Then, it is brought into a gelled state and the procedure is completed and continue by filling step.

## Claims

1. A topical pharmaceutical composition comprising, flurbiprofen or a pharmaceutically acceptable salt thereof and methyl salicylate **characterised in that** said composition comprise dimethyl sulfoxide and one or more gelling agent.

2. The topical pharmaceutical composition according to claim 1, wherein the gelling agents are selected from group comprising hydroxypropyl cellulose, hydroxyethyl cellulose, methyl cellulose, carboxymethyl cellulose, carbomer, carbomer copolymers, gelatin, aluminum monostearat, dextrin, sodium alginate, pectin and mixtures thereof.

3. The topical pharmaceutical composition according to claim 1 and 2, wherein the gelling agents are preferably hydroxypropyl cellulose and carbomer or mixtures thereof.

4. The topical pharmaceutical composition according to claim 3, wherein the weight ratio of hydroxypropyl cellulose to carbomer is from 10:1 to 1:10 by weight, preferably it is from 4:1 to 1:4 by weight of the total composition.

5. The topical pharmaceutical composition according to claim 1, wherein the amount of dimethyl sulfoxide is from 0.50 to 30.0 % by weight of the total composition, preferably it is 5.0 to 20.0 % by weight of the total composition.

6. The topical pharmaceutical composition according to any of the preceding claims, wherein it is in the form of gel, ointment, cream, spray or lotion, preferably it is in the form of gel.

7. The topical pharmaceutical composition according to any of the preceding claims, further comprising menthol.

8. The topical pharmaceutical composition according to claim 7, wherein the amount of menthol is between 0.10 to 15.0 % by weight of the total composition, preferably it is 1.0 to 10.0 % by weight of the total composition.

9. The topical pharmaceutical composition according to any of the preceding claims, further comprising surface active agents, viscosity enhancers, dissolving solvents, preservatives, antioxidants and their mixtures.

10. The topical pharmaceutical composition according to claim 9, wherein the surface active agent is polysorbate.

11. The topical pharmaceutical composition according to claim 10, wherein the amount of polysorbate is 0.05 to 5.0 % by weight of the total composition, preferably it is 0.10 to 3.0 % by weight of the total composition.

12. The topical pharmaceutical composition according to claim 9, wherein the viscosity enhancer is glycerin.

13. The topical pharmaceutical composition according to claim 12, wherein the amount of glycerin is 1.0 to 50.0 % by weight of the total composition, preferably it is 5.0 to 15.0 % by weight of the total composition.

14. The topical pharmaceutical composition according to any of the preceding claims, comprising,
| | | |
|---|---|---|
| a. | flurbiprofen | 0.50 to 15.0 % by weight |
| b. | methyl calicylate | 1.0 to 20.0 % by weight |
| c. | hydroxypropyl cellulose | 0.05 to 10.0 % by weight |
| d. | carbomer | 0.01 to 5.0 % by weight |
| e. | dimethyl sulfoxide | 0.5 to 30.0 % by weight |
| f. | polyethylene glycol | 1.0 to 50.0 % by weight |
| g. | menthol | 0.10 to 15.0 % by weight |
| h. | polysorbate | 0.05 to 5.0 % by weight |
| i. | glycerin | 1.0 to 50.0 % by weight |
| j. | purified water | 1.0 to 30.0 % by weight |
| k. | ethyl alcohol | 1.0 to 50.0 % by weight |
| l. | NaOH / HCl | pH (5.5 ±1.0) |

15. Method for preparing the topical pharmaceutical composition according to any of the preceding claims, comprising the steps of;
a. Adding carbomer, purified water, glycerin, polyethylene glycol, dimethyl sulfoxide and swelling this mixture under stirring so as to yield the first mixture,
b. adding NaOH / HCl to the first mixture to adjust the pH and stirring,
c. adding and dissolving flurbiprofen and methyl calicylate into alcohol in a separate container and then adding menthol, polysorbate and hydroxypropyl cellulose into this mixture so as to give the second mixture,
d. adding the second mixture into the first mixture under stirring.

16. The topical pharmaceutical composition according to any previous claims, for use in the treatment of pain and inflammatory symptoms associated with muscle-skeletol system, joint and soft-tissue disorders.
